# EUROPEAN PATENT APPLICATION

(11) **EP 0 897 722 A1**
(43) Date of publication of application: **24.02.1999**
(21) Application number: 98306558.2
(22) Date of filing: 18.08.1998
(51) Int. Cl.: A61K 31/445, A61K 31/38

(54) **Benzo(b)thiophene derivatives for inhibiting fibrous inflammatory diseases and Riedel's thyroiditis**

(30) Priority: 21.08.1997 US 56214 P
(71) Applicant: ELI LILLY AND COMPANY, Indianapolis, Indiana 46285 (US)
(72) Inventor: Dodge, Jeffrey Alan, Indianapolis, Indiana 46236 (US)
(74) Representative: Denholm, Anna Marie

(57) **Abstract**

The preparation of pharmaceutical formulations for inhibiting Riedel's thyroiditis and fibrous inflammatory disease in a human using 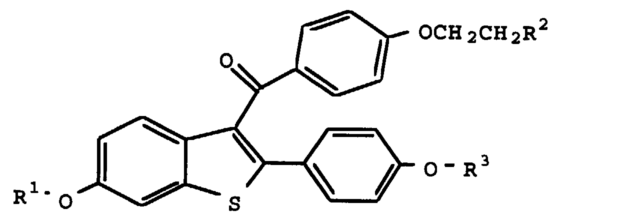 wherein R¹ and R³ are, independently, -H, -CH₃, -CO(C₁-C₆ alkyl), or -COAr, where Ar is optionally substituted phenyl;
R³ is selected from the group consisting of pyrrolidine, piperidine, and hexamethyleneimino; or
a pharmaceutically acceptable salt or solvate thereof.

## Description

The current invention deals with the areas of medicinal chemistry, pharmacology and clinical medicine related to the inhibition of Riedel's thyroiditis and fibrous inflammatory disease.

Riedel's thyroiditis is an uncommon disease; however, it is a serious and often life-threatening condition. Riedel's thyroiditis is characterized by a fibroblastic proliferation which produces a fibrous mass beginning in the thyroid and invading the surrounding tissue. The fibrous mass in this lesion consists mostly of extra-cellular matrix, mostly collagen, produced by fibroblasts. This invading fibrous lesion crowds out and destroys surrounding tissue in its path. In the case of thyroiditis, the surrounding tissues often destroyed are the airways to the lungs, the arteries to the brain, and the nerves in the neck. Thus, with the critical importance of the tissues at risk with this disease, it is not surprising that this disease is a serious medical problem. This disease is most common in women, although it is found in both sexes.

The etiology of this condition is not well understood. It may be related to an autoimmune disease or a primary fibrotic disorder. Histologic examination of the lesions indicates that Riedel's thyroiditis is in the same family of disorders categorized as inflammatory fibrous syndromes, which include the extracervical diseases, such as sclerosing cholangitis, retroperitoneal fibrosis, and the like.

The most common current therapy for treatment of Riedel's thyroiditis is surgical removal, which is often complicated by the fact that the fibrosis has invaded tissues which are not able to be removed or resectioned. Thus, surgery is often only partially successful, leaving the patient with a less than satisfactory outcome. Likewise, radiation therapy used to stop the proliferation is unsuccessful in most cases. Since halting the progression of the disease is usually not possible, treatment is aimed at keeping major organs functioning, e.g., the airway, arteries, nerves, etc. An improved therapy would be one in which the intertwined fibrous tissue would be irradicated while the normal tissue would not.

Recently, a potential new therapy has been demonstrated, see: *Riedel's Thyroiditis: Treatment with Tamoxifen,* Few, J., *et al., Surgery, (12),* pp. 993-999 (1996); and references cited therein, which are incorporated herein by reference. The success of this treatment has been attributed to the action of tamoxifen in controlling the growth factor, TGF-β. However, tamoxifen is known to have substantial estrogenic activity, which may cause uterine disorders in women and make its use in men more problematic.

The current invention provides methods for inhibiting fibrous inflammatory disease or Reidel's thyroiditis in a human, which comprises the administration to a human in need thereof of an effective amount of a compound of formula I 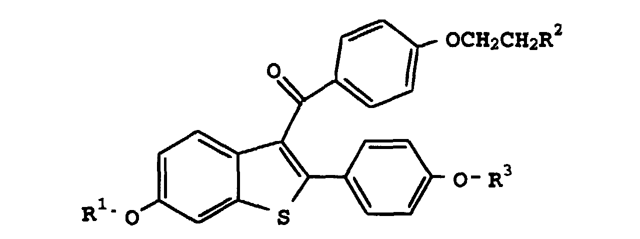 wherein
R¹ and R³ are, independently, -H, -CH₃, -CO(C₁-C₆ alkyl), or -COAr, where Ar is optionally substituted phenyl;
R³ is selected from the group consisting of pyrrolidine, piperidine, and hexamethyleneimino; or
a pharmaceutically acceptable salt or solvate, thereof.

The current invention is related to the discovery that a select group of 2-aryl benzo[b]thiophenes (the compounds of formula I) are useful inhibiting Riedel's thyroiditis and fibrous inflammatory disease.

General terms used in the description of compounds herein described bear their usual meanings. For example, "C₁-C₆ alkyl" refers to straight or branched aliphatic chains of 1 to 6 carbon atoms including methyl, ethyl, propyl, iso-propyl, n-butyl, pentyl, hexyl and the like.

The term "substituted phenyl" refers to a phenyl group alone or having one or more substituents selected from the group consisting of C₁-C₄ alkyl, C₁-C₄ alkoxy, hydroxy, nitro, chloro, fluoro, or tri(chloro or fluoro)methyl. "OC₁-C₄ alkyl" refers a C₁-C₄ alkyl group attached through an oxygen bridge such as , methoxy, ethoxy, n-propoxy, iso-propoxy, and the like.

The term "pharmaceutically acceptable salt" refers to either acid or base addition salts which are known to be non-toxic and are commonly used in the pharmaceutical literature. Commonly used acid addition salts are inorganic salts formed by the addition of sulfuric acid, nitric acid, hydrochloric acid, hydrobromic acid phosphoric acid, phosphorous acid and the like; or organic salts formed by the addition of acetic acid, formic acid, benzoic acid, citric acid, methanesulfonic acid and the like. Commonly used basic addition salts are the salts formed by alkali or alkaline earth hydroxides, ammonium hydroxide, alkyl or aromatic amines and the like. A preferred salt of this invention is the hydrochloride salt.

The term "solvate" refers to a molecular complex of a compound of formula I with one or more solvent molecules. Such solvent molecules would be those commonly used in the pharmaceutical literature, which are known to be innocuous to the recipient, e.g., water, ethanol, and the like.

The term "inhibit" is defined to include its generally accepted meaning which includes stopping, slowing, preventing or ameliorating. As such, the invention encompasses both medical therapeutic and phropholactic regimens.

The compounds of this invention are derivatives of centrally located carbon, i.e., the "-CO-" moiety in formula I, thus derivatives are methanones, e.g., a compound of A-CO-B, would be named [A][B]methanone. Further the compounds of formula I are derivatives of benzo[b]thiophene which is named and numbered according to the Ring Index, The American Chemical Society, as follows:

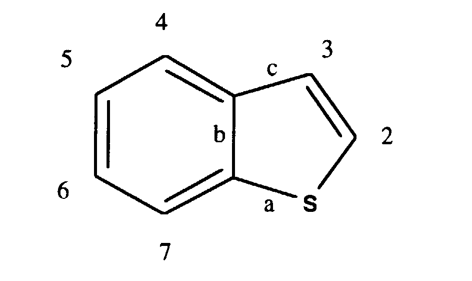

Thus, raloxifene hydrochloride, which is a preferred embodiment of this invention, is a compound of formula I, where R¹ and R³ are both hydrogen and R² is a piperidinyl ring, the hydrochloride salt thereof. Raloxifene hydrochloride is named [2-(4-hydroxyphenyl)-6-hydroxybenzo[b]thie-3-yl] [4-[2-(1-piperidenyl)ethoxy]phenyl]methanone hydrochloride.

All of the compounds used in the methods and formulations of the current invention can be made according to procedures such as those detailed in US Pat. No. 4,133,814 and US Pat. No. 4,418,068, each of which is incorporated by reference herein. In general, the process starts with a benzo[b]thiophene having a 6-hydroxyl group and a 2-(4-hydroxylphenyl) group. The starting compound is protected, alkylated, and deprotected to form the compounds of formula I. The formula I compounds which are carboxylic esters may be prepared by methods described in US Pat. No. 5,393,763, which included by reference, herein.

As used herein, the term "effective amount" means an amount of compound of the present invention which is capable of inhibiting Riedel's thyroiditis or fibrous inflammatory disease in a human.

By "pharmaceutically acceptable formulation" it is meant that the carrier, diluent, solvent, excipients and salt must be compatible with the active ingredient (a compound of formula I) of the formulation, and not be deleterious to the recipient thereof.

The compounds of formula I are members of a group of compounds known as anti-estrogens which have selective estrogenic agonist and antagonist pharmacologic activities. For example, formula I compounds act as estrogen agonists in treating pathologic sequelae caused by the cessation of menses in females (see: Draper et *al.,* "Effects of Raloxifene (Lyl39481 HC1) on Biochemical Markers of Bone and Lipid Metabolism in Healthy Postmenopausal Women", Hong Kong, Fourth Int'l. Symp. on Osteoporosis, March 29, 1993.; US Pat. Nos.5,393,763, 5,464,845, and 5,391,557).

The compounds of formula I have been shown to regulate the genes of the TGF-β family, see: Yang, N.N., *et al.,* Science, **1996**, 273, p. 1222 and references cited therein, which are included by reference herein. Additionally, the compounds of formula I have shown activity in other types of fibrotic states, see: US Pat. Nos. 5,457,116 and 5,574,047.

Pharmaceutical formulations can be prepared by procedures known in the art. For example, the compounds of this invention can be formulated with common excipients, diluents, or carriers, and formed into tablets, capsules, and the like. Examples of excipients, diluents, and carriers that are suitable for such formulations include the following: fillers and extenders such as starch, sugars, mannitol, and silicic derivatives; binding agents such as carboxymethyl cellulose and other cellulose derivatives, alginates, gelatin, and polyvinyl pyrrolidone; moisturizing agents such as glycerol; disintegrating agents such as agar agar, calcium carbonate, and sodium bicarbonate; agents for retarding dissolution such as paraffin; resorption accelerators such as quaternary ammonium compounds; surface active agents such as cetyl alcohol, glycerol monostearate; adsorptive carriers such as kaolin and bentonire; and lubricants such as talc, calcium and magnesium stearate and solid polyethyl glycols. Final pharmaceutical forms may be pills, tablets, powders, lozenges, syrups, aerosols, saches, cachets, elixirs, suspensions, emulsions, ointments, suppositories, sterile injectable solutions, or sterile packaged powders, depending on the type of excipient used.

Additionally, the compounds of this invention are well suited to formulation as sustained release dosage forms. The formulations can also be so constituted that they release the active ingredient only or preferably in a particular part of the intestinal tract, possibly over a period of time. Such formulations would involve coatings, envelopes, or protective matrices which may be made from polymeric substances or waxes.

The particular dosage of a compound of formula I required to inhibit Riedel's thyroiditis or fibrous inflammatory disease according to this invention will depend upon the particular symptom and severity. Such considerations as dosage, route of administration, and frequency of dosing are best decided by the attending physician. Generally, accepted and effective doses for oral or parenteral administration will be from 10mg to 800mg, and more typically between 20mg and 100mg. A particularly preferred dose is 60 mg/day via the oral route, especially in a post-menopausal female. Such dosages will be administered to a patient in need of treatment from once to three times each day or as often as needed to effectively address the malady.

The formulations which follow are given for purposes of illustration and are not intended to be limiting in any way. The total active ingredients in such formulations comprises from 0.1% to 99.9% by weight of the formulation. The term, "active ingredient" means a compound of formula I, preferably Raloxifene hydrochloride.

| Formulation 1: Gelatin Capsules | |
|---|---|
| Ingredient | Quantity (mg/capsule) |
| Active Ingredient | 50-600 |
| Starch NF | 0-500 |
| Starch flowable powder | 0-500 |
| Silicone fluid 350 centistrokes | 0-15 |

The ingredients are blended, passed through a No. 45 mesh U.S. sieve, and filled into hard gelatin capsules.

| Formulation 2: Tablets | |
|---|---|
| Ingredient | Quantity (mg/tablet) |
| Active Ingredient | 50-600 |
| Starch | 10-50 |
| Cellulose, microcrystalline | 10-20 |
| Polyvinylpyrrolidone (as 10% solution in water) | 5 |
| Sodium carboxymethyl cellulose | 5 |
| Magnesium stearate | 1 |
| Talc | 1-5 |

The active ingredient, starch, and cellulose are passed through a No. 45 mesh U.S. sieve and mixed thoroughly. The solution of polyvinylpyrrolidone is mixed with the resultant powders which are then passed through a No. 14 mesh U.S. sieve. The granules thus produced are dried at 50-60° C and passed through a No. 18 mesh U.S. sieve. The sodium carboxymethyl cellulose, magnesium stearate, and talc, previously passed through a No. 60 mesh U.S. sieve, are added to the above granules and thoroughly mixed. The resultant material is compressed in a tablet forming machine to yield the tablets.

| Formulation 3: Aerosol | |
|---|---|
| Ingredient | Weight % |
| Active Ingredient | 0.50 |
| Ethanol | 29.50 |
| Propellant 22 (Chlorodifluoromethane) | 70.00 |
| | |
| Total | 100.00 |

The active ingredient is mixed with ethanol and the mixture added to a portion of the propellant 22, cooled to -30°C and transferred to a filling device. The required amount is then fed to a stainless steel container and diluted with the remainder of the propellant. The valve units are then fitted to the container.

| Formulation 4: Suspension | |
|---|---|
| Suspensions each containing 100 mg of a compound of formula I per 5 mL dose. | |

| Ingredient | Weight |
|---|---|
| Active Ingredient | 100 mg |
| Sodium carboxymethyl cellulose | 50 mg |
| Syrup | 1.25 mL |
| Benzoic acid solution (0.1M) | 0.10 mL |
| Flavor | q.v. |
| Color | q.v. |
| Purified water to total | Total 5 mL |

A compound of formula I is passed through a No. 45 mesh U.S. sieve and mixed with the sodium carboxymethyl cellulose and syrup to form a smooth paste. The benzoic acid solution, flavor, and color diluted in water are added and mixture stirred thoroughly. Additional water is added to bring the entire mixture to the required volume.

Any patient suffering from fibrous inflammatory disease may be a candidate for such a demonstration, but for the purposes of illustration, the example of a patient suffering from Riedel's thyroiditis is presented.

A person who is suffering from Riedel's thyroiditis is selected. Such a patient is screened by histrological examination of biopsy tissue of the thyroid lesion in order to determine a definite diagnosis of Riedel's thyroiditis. In addition, the biopsy tissue will be examined for the presence of estrogen receptors (patients should demonstrate some level of estrogen receptor for best results), and TGF-β. The patient's symptoms are also recorded, e.g., physical activity capacity, ease of breath, ease of eating, emotional state, etc. Further, CT scans of the effected areas are taken and evaluated. These parameters are gathered and recorded for the initial time point and at subsequent time intervals of six months. The patient is then administered 60 mg of Raloxifene hydrochloride per day via an oral formulation. This therapy will continue for a period of three years with an evaluation of the parameters, *supra,* every six months. A decrease in the fibroid mass as evaluated by the CT scans and an improvement in symptoms, e.g., easier breathing, eating, etc., indicates a positive response by the patient to the methods of the current invention.

## Claims

1. The use of a compound of formula I 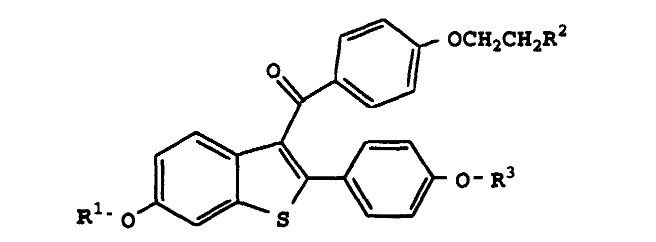 wherein
R¹ and R³ are, independently, -H, -CH₃, -CO(C₁-C₆ alkyl), or -COAr, where Ar is optionally substituted phenyl;
R³ is selected from the group consisting of pyrrolidine, piperidine, and hexamethyleneimino; or
a pharmaceutically acceptable salt or solvate thereof, in the preparation of a pharmaceutical formulation for inhibiting Riedel's thyroiditis in a human.

2. A use according to Claim 1 wherein said compound is [2-(4-hydroxyphenyl)-6-hydroxybenzo[b]thien-3-yl][4-[2-(1-piperidinyl)ethoxy]phenyl]methanone hydrochloride.

3. A use of Claim 1 wherein said human is a woman.

4. A use according to Claim 2 wherein compound is administered in an amount of 60 mg/day via the oral route.

5. The use of a compound of formula I 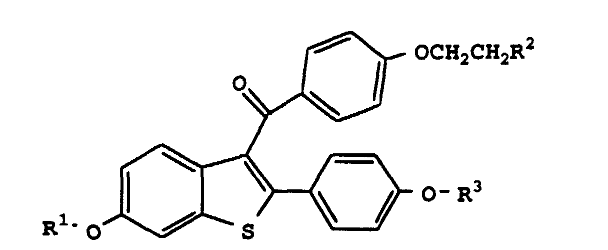 wherein
R¹ and R³ are, independently, -H, -CH₃, -CO(C₁-C₆ alkyl), or -COAr, where Ar is optionally substituted phenyl;
R³ is selected from the group consisting of pyrrolidine, piperidine, and hexamethyleneimino; or
a pharmaceutically acceptable salt or solvate thereof, in the preparation of a pharmaceutical formulation for inhibiting fibrous inflammatory disease in a human.

6. A use according to Claim 5 wherein said compound is [2-(4-hydroxyphenyl)-6-hydroxybenzo[b]thien-3-yl][4-[2-(1-piperidinyl)ethoxy]phenyl]methanone hydrochloride.

7. A use of Claim 5 wherein said human is a woman.

8. A use according to Claim 6 wherein compound is administered in an amount of 60 mg/day via the oral route.
